Europäisches Patentamt

European Patent Office

Office européen des brevets

(11) Publication number: **0 083 224 B1**

# EUROPEAN PATENT SPECIFICATION

(45) Date of publication of patent specification: 27.05.87

(21) Application number: 82306925.7

(22) Date of filing: 23.12.82

(51) Int. Cl.⁴: **C 07 C 51/265,** C 07 C 51/487, C 07 C 63/307, C 07 C 63/313

(54) Process for producing aromatic polycarboxylic acid with high purity.

(30) Priority: 28.12.81 JP 211903/81

(43) Date of publication of application:
06.07.83 Bulletin 83/27

(45) Publication of the grant of the patent:
27.05.87 Bulletin 87/22

(84) Designated Contracting States:
DE GB IT

(56) References cited:
DE-A-2 743 004
GB-A-1 152 576
GB-A-2 051 039
GB-A-2 056 979

(73) Proprietor: MITSUBISHI GAS CHEMICAL
COMPANY, INC.
5-2, Marunouchi 2-chome Chiyoda-Ku
Tokyo (JP)

(72) Inventor: Tanaka, Toru
400-50 Fukui
Kurashiki-shi (JP)
Inventor: Hataya, Masanori
66-5, Nishinakashinden
Kurashiki-shi (JP)
Inventor: Tanaka, Kazuo
400-50 Fukui
Kurashiki-shi (JP)

(74) Representative: Baillie, Iain Cameron et al
c/o Ladas & Parry Isartorplatz 5
D-8000 München 2 (DE)

Courier Press, Leamington Spa, England.

## Description

The present invention relates to a process for producing an aromatic polycarboxylic acid anhydride with high purity by oxidizing a polyalkyl-substituted aromatic aldehyde or a polyalkyl-substituted aromatic carboxylic acid in water as a solvent in the presence of a bromine ion-containing catalyst.

Among aromatic polycarboxylic acids, trimellitic acid is widely used as raw materials for alkyd resin, high grade plasticizer, polyamide-imide polyester, and pyromellitic acid is used as raw materials for special plasticizer, polyimide and crosslinking agent.

However generally high purity is required for the aromatic polycarboxylic acids for such uses. For example, trimellitic acid must have a purity of higher than 99%, and a TEG color test value of less than 170 (TEG color test value designates a coloring degree where a smaller TEG value means less content of coloring matters).

The so far well known processes for producing trimellitic acid in a commercial scale include (i) a process by oxidizing pseudocumene as a raw material with air at 1 to 3 stages in acetic acid as a solvent in the presence of a Co—Mn—Br catalyst and (ii) a process by oxidizing pseudocumene as a raw material with nitric acid likewise. Recently, (iii) a process by oxidizing dimethylbenzaldehyde with air in water as a solvent in the presence of bromine and a metal catalyst (British Patent No. 2,056,979).

These three processes (i)—(iii) require different raw materials, solvents, catalysts and oxidizing agents, and thus produce quite different impurities as by-products, though the same trimellitic acid is the desired product.

In the process (i), the main inpurities are tetra or pentacarboxylic acid of condensed two nuclei and tarry high molecular weight substances. In the process (ii), they are nitro compounds originating from the nitric acid as the oxidizing agent. In the process (iii), on the other hand bromine compounds are produced as impurities. Thus, different purification means are required for the production of trimellitic acid with high purity, depending upon the individual processes.

On the other hand, a somewhat similar process for purification is disclosed (Japanese Patent Publication No. 16860/66 in the name of Standard Oil Company and published September 24, 1966), where fiber grade terephthalic acid is produced by treating an impure aqueous terephthalic acid solution containing a large amount of 4-carboxybenzaldehyde and coloring impurities, as obtained by catalytic liquid phase oxidation of paraxylene with molecular oxygen, with a reducing agent in the most preferable temperature range of 225° to 275°C. However, the desired effect of purification cannot be obtained by applying the process as such to the treatment of the bromine compounds produced as by-products in the process (iii), for most of trimellitic acid rather than the bromine compounds is further converted to methylphthalic acid, phthalic acid and even to toluic acid under the said hydrogenating conditions, which lead to considerable deterioration of distillation efficiency and product purity.

GB—A—1152576 discloses a process for purifying an aromatic polycarboxylic acid and in particular terephthalic acid, (as obtained by oxidation in the presence of a heavy metal of p-xylene), and subsequently by hydrogen treatment in the presence of a catalyst containing a platinum group metal acid is once isolated, dissolved in water, and subjected to the hydrogen treatment (page 3, lines 1—3), and the heating temperature is 392°—700°F (200°—370°), which is different from that of the present invention. Furthermore, in GB—A—1,152,576, the hydrogen treatment is carried out to treat 4-carboxybenzaldehyde contained in the product terephthalic acid with hydrogen to purify terephthalic acid (page 2, lines 125—130). In the present invention, on the other hand, benzene ring compounds nuclearly substituted with bromine are decomposed.

G.B. 2,051,039 discloses a process of purifying terephthalic acid by dissolving crude terephthalic acid in water, hydrogenating the aqueous solution of terephthalic acid with molecular hydrogen in the presence of a hydrogenation catalyst, and recrystallizing the terephthalic acid from the hydrogenated aqueous solution where an alkali material is added to the aqueous solution of terephthalic acid to be purified before the hydrogenation.

As a result of further studies or reaction selectively in hydrogenation of trimellitic acid and bromine compounds, the present inventors have found that, by conducting hydrogenation at a hydrogenation temperature of 100° to 180°C the bromine compounds can be removed while preventing hydrogenating or trimellitic acid, and have established the present invention.

Summary of the invention

The present invention provides a process for producing an aromatic polycarboxylic acid anhydride with high purity, which comprises oxidizing a polyalkyl-substituted aromatic aldehyde or polyalkyl-substituted aromatic carboxylic acid with molecular oxygen in water as a solvent in the presence of bromine ion or bromine ion and heavy metal ion as a catalyst contacting the oxidation reaction product with molecular hydrogen in the presence of a hydrogenating catalyst at a temperature of 100° to 180°C, and then separating an aromatic polycarboxylic acid from the hydrogenated product.

Where the aromatic aldehydes are oxidized in the presence of bromine catalyst in water as a solvent, there takes place a peculiar phenomenon in that benzene ring compounds nuclearly substituted with bromine are formed. These impurities are removed by hydrogenation of reaction products.

Such benzene ring compounds nuclearly substituted with bromine are not formed in the oxidation reaction in acetic acid as a solvent. The present invention is based on a finding that such compounds nuclearly substituted with bromine can be decomposed and removed by hydrogenation at 100°—180°C while preventing hydrogenation of carboxylic acid.

Hydrogenation at 100°—180°C is very important for the effect of the present invention. Without the hydrogenation, the bromine content of the product aromatic polycarboxylic acid is a few 1,000 ppm, which cannot attain the object of the present invention cannot attain the object of the present invention. When the hydrogenation temperature is outside 100°—180°C the bromine content and TEG color of the product are higher and the product purity and distillation yield are low. TEG color is an APHA number index of the color of heated solution consisting of 4.0g of trimellitic anhydride and 30.0g of triethylene glucol, where a small value of TEG color means less content of coloring matters;

The polyalkyl-substituted aromatic aldehyde to be used as the raw material in the oxidation reaction according to the present invention includes 2,4-dimethylbenzaldehyde, 3,4-dimethylbenzaldehyde, 2,4,5-trimethylbenzaldehyde, 2,4,6-trimethylbenzaldehyde, etc., which are correspondingly oxidized to trimellitic acid, pyromellitic acid, mellophannic acid, etc.

The polyalkyl-substituted aromatic aldehyde can be stoichiometrically synthesized without any production of isomers as by-product by reacting a polyalkl benzene with carbon monoxide in the presence of a catalyst of HF—$BF_3$ system.

In the present invention, polyalkyl-substituted aromatic carboxylic acid can be likewise used as a raw material for the oxidation reaction. Examples of the polyalkyl-substituted carboxylic acid include 2,4-dimethylbenzoic acid, 3,4-dimethylbenzoic acid, 2,4-5-trimethylbenzoic acid, 2,4,6-trimethylbenzoic acid, etc., which are correspondingly oxidized to trimellitic acid, pyromellitic acid, mellophannic acid, etc.

Bromine ion can be used as the catalyst in the oxidation reaction, but it is preferable to use bromine ion together with heavy metal ion. Particularly preferable heavy metal ion is manganese ion and cerium ion. Some heavy metals such as palladium, ruthenium, bismuth, niobium, thallium, tellurium, vanadium, etc. deteriorate the catalytic activity of bromine ions, and cannot be used as the catalyst. A bromine ion-liberating compound can be also used, so far as it can liberate bromine ions in the course of oxidation reaction. For example, hydrogen bromide, ammonium bromide, sodium bromide, or organobromo compounds such as alkyl bromide, etc. can be used.

The amount of bromine ions as the catalyst is 0.5 to 12% by weight, preferably 0.5 to 6% by weight on the basis of water as the solvent. The amount of the heavy metal ion as the catalyst in the equivalent weight or less than the equivalent weight to that of the bromine ion. The amount of water as the solvent is not particularly limited, but preferably is at least equal to the weight of the aldehyde or the carboxylic acid as the raw material.

In the present invention, oxidation reaction temperature is 180°—280°C, preferably 200°C—260°C. Oxidation reaction pressure is automatically set by keeping the reaction temperature constant generally by evaporation and condensation and refluxing operation of water as the solvent, but it is also possible to keep the oxidation reaction pressure at a desired value by the external heat exchanger. Any pressure can be applied so far as it is within a pressure range in which the reaction solution can be kept in a liquid phase, and usually a pressure 15—60kg/cm² gage is used.

The oxidation reaction can be carried out batchwise, semi-continuously, or continuously, but the best effect can be obtained particularly when the present invention is applied to the conventional continuous oxidation process requiring a plurality of reactors at a plurality of stages.

The reaction product from the oxidation reaction is subjected to cooling, crystallization and solid-liquid separation, and then the resulting crude aromatic polycarboxylic acid is dissolved in water, and then the aqueous solution is supplied to a hydrogenating reactor, or the reaction product as such is supplied to the hydrogenating reactor without any solid-liquid separation. No such solid-liquid separation is required under such an oxidation condition to produce less by-products of bromine compounds as impurities. The crude aqueous polycarboxylic acid solution to be hydrogenated usually has a polycarboxylic acid concentration of 15 to 50% by weight, and it is desirable to supply the crude aqueous solution at a lower polycarboxylic acid concentration in the case of a higher content of bromine compounds in the case of a larger amount of bromine ions being used as the oxidation catalyst. The preferable concentration of bromine ion is 0.2—1.0% by weight in the hydrogenating reaction.

The hydrogenation catalyst includes catalysts containing at least one of noble metal species belonging to group 8 of the periodic table, for example, Pd, Pt, Ru, Rh, etc. These noble catalysts can be used in any form, for example, simple substance, alloy, mixture or carrier-supported catalyst, preferably an activated carbon-supported catalyst. The carrier may be in a powder form or in a pellet form, but the pellet form is often advantageous for continouus operation because of its applicability to a fixed bed.

Hydrogenation temperature is 100°—180°C. Hydrogen pressure is high enough to keep the crude aqueous polycarboxylic acid solution in a liquid state, and is usually 5—30kg/cm² gage, preferably 5—20kg/cm² gage. Hydrogenation time depends upon the concentration of bromine compounds as impurities, hydrogenation temperature, the amount of catalyst, the catalyst activity and the desired purity of product polycarboxylic acid, and is usually 0.1 to 8 hours, preferably 0.2 to 3 hours.

The catalyst containing 0.5% by weight of a noble metal catalyst supported on a carrier on the basis of the carrier is used in an amount of 0.05 to 5.0% by weight on the basis of polycarboxylic acid for batchwise hydrogenation under the said hydrogenation conditions.

The hydrogenation can be carried out batchwise, semi-batchwise, or continuously. According to the conventional process, the hydrogenation product mixture is then subjected to dehydration by heating, and the resulting anhydrous mixture is then subjected to distillation under a subatmospheric pressure to obtain an anhydrous aromatic polycarboxylic acid product.

According to the present invention, an aromatic polycarboxylic acid anhydride with high purity can be readily obtained from the crude aromatic polycarboxylic acid obtained by oxidation of a polyalkyl-substituted aromatic aldehyde or polyalkyl-substituted aromatic carboxylic acid in the presence of bromine ion as a catalyst.

Preferred embodiments of the invention

The present invention will be described in detail below, referring to Examples, where the bromine content is a content of bromine compounds in terms of bromine determined by X-ray fluorometry analysis on the basis of trimellitic acid; TEG color is an APHA number index of the color of heated solution consisting of 4.0g of trimellitic anhydride and 30.0g of triethylene glycol, where a smaller value of TEG color means less content of coloring matters; purity is a percentage of the acid value of product trimellitic acid to the acid value of pure trimellitic acid; and distillation yield is a percentage of the amount of main fraction as a distillate, to the amount of it in a feedstock to the distillation.

Example 1
(Direct hydrogenation without solid-liquid separation)

Into an autoclave made from zirconium having a net capacity of 2 with a reflux condenser, a stirrer, a heater, a raw material inlet, a gas inlet, a gas outlet and a product outlet were charged 500g of water, 15g of manganese bromide tetrahydrate, and 7g of hydrogen bromide. Nitrogen was introduced under pressure into the autoclave at the gas inlet to elevate the inside pressure of the autoclave to 10kg/cm² gage. The autoclave was heated to 220°C by a heater and then 2,4-dimethylbenzaldehyde having a purity of at least 99.5% was introduced into the autoclave at a rate of 4.17g/min. At the same time, air was introduced into the autoclave at a controlled flow rate to keep the oxygen concentration of the effluent gas from the autoclave at 3-4% by volume. 2,4-dimethylbenzaldehyde was continuously introduced into the autoclave for 60 minutes, while air was supplied, even after the introduction of 2,4-dimethylbenzaldehyde was finished, to the autoclave for further 20 minutes to complete oxidation. The oxidation product was cooled to 20°C, and then taken out of the autoclave. The oxidation product had a bromine content of 6,500 ppm and had a purity of 95.6% by weight in terms of trimellitic acid.

970.5g of the oxidation product containing 356.5g of trimellitic acid was charged into an autoclave having a net capacity of 2 l, provided with a catalyst basket with 1g of a 0.5wt.% Pd-C catalyst, and then the autoclave was pressurized with a hydrogen gas under a pressure of 15kg/cm² gage, and then heated at 150°C for 2 hours while injecting the hydrogen gas into the liquid phase and passing it through the autoclave to conduct hydrogenation. Then, the autoclave was slowly cooled to 20°C, and the precipitated crystal was recovered by filtration and dried. The thus obtained trimellitic acid was placed into a distilling still and heated at 230°C to 240°C under a pressure of 50 to 100 mmHg (absolute) to conduct dehydration, and then distilled at 230° to 240°C under a pressure of 4 to 5 mmHg (absolute) to obtain trimellitic anhydride. The thus obtained trimellitic anhydride had a purity of 99.5%, a bromine content of 150 ppm, a TEG color value of 60 and a distillation yield of 97.5%.

Comparative Example 1

The oxidation product as obtained in Example 1 was slowly cooled to 20°C without hydrogenation, and 358g of the precipitated crude trimellitic acid crystal was placed in a distilling still, and heated at 230°—240°C under a pressure of 50—100mmHg (absolute) to conduct dehydration and then distilled at 230°—240°C under a pressure of 4—5 mmHg (absolute) to obtain trimellitic anhydride. The resulting trimellitic anhydride had a purity of 96.5%, a bromine content of 5,000ppm, TEG color value of 800 and a distillation yield of 93.2%.

Comparative Example 2

The reaction product as obtained in Example 1 was slowly cooled to 20°C without hydrogenation, and 358g of the precipitated crude trimellitic acid crystal was separated and admixed with 1,100g of water. Then, the mixture was heated to 100°C to dissolve the crude trimellitic acid crystal. Then, the solution was cooled to 20°C, and the precipitated crystal was recovered by filtration, and subjected to dehydration and distillation in the same manner as in Example 1 to obtain trimellitic anhydride. The thus obtained trimellitic anhydride had a purity of 97.0%, a bromine content of 4,500ppm, a TEG color value of 750, and a distillation yield of 94.0%.

Example 2
(Solid-liquid separation and hydrogenation)

The oxidation product as obtained in Example 1 was cooled slowly to 20°C, and 358g of the precipitated crystal was separated and admixed with 550g of water. The resulting slurry was charged into an autoclave having a 2-l capacity, provided with a catalyst basket with 1g of 0.5% Pd/C catalyst. Then, the autoclave was pressurized with a hydrogen gas under a pressure of 15kg/cm² gage and then heated at 150°C for 1.5 hours, while passing the hydrogen gas through the autoclave to conduct hydrogenation. Then, the autoclave was slowly cooled to 20°C, and the precipitated crystal was recovered by filtration and dried. Then, the resulting trimellitic acid was placed in a distilling still and subjected to dehydration and distillation in the same manner as in Example 1 to obtain trimellitic anhydride. The resulting trimellitic anhydride had a purity of 99.7%, a bromine content of 70 ppm, a TEG color value of 40, and a distillation yield of 98.0%.

Comparative Example 3

When the crude trimellitic acid crystal was dissolved in Comparative Example 2, 20g of activated carbon granules was added to the solution. The resulting mixture was kept at 100°C with stirring for 60 minutes and then filtered, while hot, to remove the activated carbon granules. The filtrate was slowly cooled to 20°C, and the precipitated crystal was recovered by fitration, and subjected to dehydration and distillation in the same manner as in Example 1 to obtain trimellitic anhydride. The resulting trimellitic anhydride had a purity of 97.2%, a bromine content of 4,100 ppm, a TEG color value of 600, and a distillation yield of 94.2%. It was found that the impurities could not be removed by adsorption with the activated carbon.

Example 3
(Solid-liquid separation and hydrogenation)

The oxidation product as obtained in Example 1 was slowly cooled to 20°C to separate 358g of precipitated crude crystal, and the crude crystal was admixed with 550g of water in the same manner as in Example 2.

Then, the resulting slurry was charged into an autoclave having a 2-l capacity, provided with a catalyst basket with 0.5g of 0.5% Pd/C catalyst, and the autoclave was pressurized with a hydrogen gas under a pressure of 25kg/cm² gage and then heated at 180°C for 30 minutes while passing the hydrogen gas through the autoclave to conduct hydrogenation. Then, the autoclave was slowly cooled to 20°C, and the precipitated crystal was recovered by filtration and dried. The resulting trimellitic acid crystal was placed in a distilling still and subjected to dehydration and distillation in the same manner as in Example 1 to obtain trimellitic anhydride. The resulting trimellitic anhydride had a purity of 99.3%, a bromine content of 100 ppm, a TEG color value of 60 and a distillation yield of 97.7%.

Example 4
(Direct hydrogenation without solid-liquid separation)

970.5g of the oxidation product as obtained in Example 1 was charged into an autoclave having a 2-l capacity, provided with a catalyst basket with 1.0g of 0.5% Pd/C catalyst, and then the autoclave was pressurized with a hydrogen gas under a pressure of 15kg/cm² gage, and heated at 120°C for 3 hours while passing the hydrogen gas through the autoclave to conduct hydrogenation. Then, the autoclave was slowly cooled to 20°C, and the precipitated crystal was recovered by filtration and dried. The resulting trimellitic acid was placed in a distilling still, and subjected to dehydration and distillation in the same manner as in Example 1 to obtain trimellitic anhydride. The resulting trimellitic anhydride had a purity of 99.3%, a bromine content of 250 ppm, a TEG color value of 90, and a distillation yield of 97.1%.

Example 5 (Direct hydrogenation without solid-liquid separation)

970.5g of the oxidation product as obtained in Example 1 was charged into an autoclave having a 2-l capacity, provided with a catalyst basket with 1.0g of 0.5% Pd/C catalyst, and then the autoclave was pressurized with a hydrogen gas under a pressure of 68kg/cm² gage, and heated at 260°C for 60 minutes while passing the hydrogen gas through the autoclave to conduct hydrogenation. Then, the autoclave was slowly cooled to 20°C, and the precipitated crystal was recovered by filtration and dried. The resulting trimellitic acid was placed in a distilling still and subjected to dehydration and distillation in the same manner as in Example 1 to obtain trimellitic anhydride. The resulting trimellitic anhydride had a purity of 92.1%, a bromine content of 750 ppm, a TEG color value of 250, and distillation yield of 83.8%.

Example 6
(Solid-liquid separation and hydrogenation)

The oxidation product as obtained in Example 1 was slowly cooled to 20°C to separate 358g of precipitated crude crystal, and the crude crystal was admixed with 550g of water in the same manner as in Example 2.

Then, the resulting slurry was charged into an autoclave having a 2-l capacity, provided with a catalyst basket with 0.5g of 0.5% Pd/C catalyst, and the autoclave was pressurized with a hydrogen gas under a pressure of 35kg/cm² gage and then heated at 220°C for 30 minutes while passing the hydrogen gas through the autoclave to conduct hydrogenation. Then, the autoclave was slowly cooled to 20°C, and the precipitated crystal was recovered by filtration and dried. The resulting trimellitic acid crystal was placed in a distilling still and subjected to dehydration and distillation in the same manner as in Example 1 to obtain trimellitic anhydride. The resulting trimellitic anhydride had a purity of 94.5%, a bromine content of 600 ppm, a TEG color value of 200 and a distillation yield of 86.0%.

Example 7
(Effect of temperature)

970.5g of the oxidation product as obtained in Example 1 was charged into an autoclave having a 2-l capacity, provided with a catalyst basket with 2.0g of 0.5% Pd/C catalyst, and then the autoclave was pressurized with a hydrogen gas under a pressure of 20kg/cm² gage, and heated at 80°C for 3 hours while passing the hydrogen gas through the autoclave to conduct hydrogenation. Then, the autoclave was slowly cooled to 20°C, and the precipitated crystal was recovered by filtration and dried. The resulting trimellitic acid was placed in a distilling still, and subjected to dehydration and distillation in the same manner as in Example 1 to obtain trimellitic anhydride. The resulting trimellitic anhydride had a purity of 98.5%, a bromine content of 400 ppm, a TEG color value of 140, and a distillation yield of 89.9%.

Comparative Example 4
The oxidation product as obtained in Example 1 was slowly cooled to 20°C to separate 358g of precipitated crude crystal, and the crude crystal was admixed with 900g of water and heated at 100°C to obtain a solution.

Then, 1.5g of iron powder and 12g of 35% hydrochloric acid were added to the solution and kept at 100°C with stirring for 3 hours. Then, the mixture was slowly cooled to 20°C, and the precipitated crystal was recovered by filtration and dried. The resulting trimellitic acid crystal was placed in a distilling still and subjected to dehydration and distillation in the same manner as in Example 1 to obtain trimellitic anhydride. The resulting trimellitic anhydride had a purity of 96.7%, a bromine content of 4,800 ppm, a TEG color value of 750 and a distillation yield of 94.2%. It was found that the reduction treatment by iron was not effective.

Example 8
500g of water and 17.5g of HBr were charged into a zirconium autoclave having a 2-l capacity, and then was pressurized with a nitrogen gas under a pressure of 10kg/cm² gage and then heated at 230°C by a heater. Then, 2,4-dimethylbenzaldehyde having a purity of at least 99.5% was supplied to the autoclave at a rate of 4.17g/min. At the same time, air was introduced into the autoclave at such a controlled flow rate as to keep the oxygen concentration of the effluent gas from the autoclave at 3—4% by volume. The 2,4-dimethylbenzaldehyde was continuously supplied to the autoclave for 60 minutes, while the air was continuously supplied thereto, even after that, for further 20 minutes to complete the oxidation. After cooling to 20°C, the oxidation reaction product was taken out. The oxidation product had a bromine content of 8,500 ppm and had a purity of 94.4% in terms of trimellitic acid.

962.8g of the oxidation product containing 333.0g of trimellitic acid was charged into an autoclave having a 2-l capacity, provided with a catalyst basket with 3g of 0.5% Pd/C catalyst, and then the autoclave was pressurized with a hydrogen gas under a pressure of 25kg/cm² gage, and heated at 140°C for 2 hours while injecting the hydrogen gas into the liquid phase and passing it through the autoclave to conduct hydrogenation. Then, the autoclave was slowly cooled to 20°C, and the precipitated crystal was recovered by filtration and dried. The resulting trimellitic acid was placed in a distilling still, and heated at 230°—240°C under a pressure of 50—100mmHg (absolute) to conduct dehydration, and then distilled at 230°—240°C under a pressure of 4—5mmHg (absolute) to obtain trimellitic anhydride. The resulting trimellitic anhydride had a purity of 99.1%, a bromine content of 240 ppm, a TEG color value of 90, and a distillation yield of 96.8%.

Example 9
The oxidation product as obtained in Example 8 was slowly cooled to 20°C to separate 336.4g of precipitated crude crystal, and the crude crystal was admixed with 550g of water.

Then, the resulting slurry was charged into an autoclave having a 2-l capacity, provided with a catalyst basket with 2g of 0.5% Pd/C catalyst, and the autoclave was pressurized with a hydrogen gas under a pressure of 15kg/cm² gage and then heated at 165°C for 1.5 hours while passing the hydrogen gas through the autoclave to conduct hydrogenation. Then, the autoclave was slowly cooled to 20°C, and the precipitated crystal was recovered by filtration and dried. The resulting trimellitic acid crystal was placed in a distilling still and subjected to dehydration and distillation in the same manner as in Example 1 to obtain trimellitic anhydride. The resulting trimellitic anhydride had a purity of 99.3%, a bromine content of 110 ppm, a TEG color value of 60, and a distillation yield of 97.3%.

**Claims**

1. A process for producing an aromatic polycarboxylic acid anhydride with a purity of higher than 99%, which comprises oxidizing a polyalkyl-substituted aromatic aldehyde or polyalkysubstituted aromatic carboxylic acid with molecular oxygen in water as a solvent in the presence of bromine ion or bromine ion and heavy metal ion as a catalyst, characterised in that the oxidation reaction product is contacted with molecular hydrogen in the presence of a hydrogenating catalyst and the absence of alkaline material at a temperature of 100°C to 180°C, the hydrogenation product mixture is subjected to dehydration by heating and the resulting anhydrous product is subjected to distillation under a subatmospheric pressure.

2. The process according to claim 1, wherein the polyalkyl-substituted aromatic aldehyde is 2,4-dimethylbenzaldehyde, 3,4-dimethylbenzaldehyde, 2,4,5-trimethylbenzaldehyde or 2,4,6-trimethylbenzaldehyde.

3. The process according to Claim 1, wherein the polyalkyl-substituted aromatic acid is 2,4-dimethylbenzoic acid, 3,4-dimethylbenzoic acid, 2,4,5-trimethylbenzoic acid, or 2,4,6-trimethylbenzoic acid.

4. The process according to Claim 1, wherein the bromine ion is liberated from hydrogen bromide, ammonium bromide, sodium bromide, or alkyl bromide.

5. The process according to Claim 1, wherein the bromine ion is used in an amount of 0.5—12% by weight on the basis of the water as the solvent.

6. The process according to claim 1, wherein the metal ions of manganese or cerium is used in equivalent amount or less than that to the bromine ion.

7. The process according to Claim 1, wherein the water is used in weight equal to or more than that of the polyalkyl-substituted aromatic aldehyde or the polyalkyl-substituted aromatic acid.

8. The process according to Claim 1, wherein the oxidation is carried out at a reaction temperature of 180°—280°C under a pressure of 15—60kg/cm² gage.

9. The process according to Claim 1, wherein the molecular oxygen is in the form of air.

10. The process according to Claim 1, wherein the hydrogenating catalyst is at least one of noble metals belonging to group 8 of the periodic table.

11. The process according to Claim 10, wherein the noble metals are Pd, Pt, Ru, and Rh.

12. The process according to Claim 1, wherein the hydrogenating catalyst is in a simple substance form, an alloy form, a mixture form, or a carrier-supported form.

13. The process according to Claim 12, wherein the carrier is activated carbon in a pellet form.

14. The process according to Claim 1, wherein the hydrogenation is carried out at 100°—200°C under a pressure of 5—30kg/cm² for 0.1—8 hours.

## Patentansprüche

1. Verfahren zum Erzeugen des Anhydrids einer aromatischen Polycarbonsäure in einer Reinheit über 99%, in dem ein polyalkylsubstituiertes aromatisches Aldehyd oder eine polyalkylsubstituierte aromatische Carbonsäure in Wasser als Lösungsmittel und in Gegenwart van Bromionen oder van Bromionen und Schwermetallionen als Katalysator mit molekularem Sauerstoff oxidiert wird, dadurch dekennzeichnet, daß das Produkt der Oxidationsreaktion in Gegenwart eines Hydrierkatalysators und in Abwesenheit von Alkali bei einer Temperatur von 100 bis 180°C mit molekularem Wasserstoff in Berührung gebracht wird, daß das Hydrierungsprodukt durch Erhitzen entwässert wird und daß das so erhaltene wasserfreie Produkt unter einem Unterdruck destilliert wird.

2. Verfahren nach Anspruch 1, dadurch gekennzeichnet, daß das polyalkylsubstituierte aromatische Aldehyd 2,4-Dimethylbenzaldehyd, 3,5-Dimethylbenzaldehyd, 2,4,5-Trimethylbenzaldehyd oder 2,4,6-Trimethylbenzaldehyd ist.

3. Verfahren nach Anspruch 1, dadurch gekennzeichnet, daß die polyalkylsubstituierte aromatische Säure die 2,4-Dimethylbenzoesäure, 3,4-Dimethylbenzoesäure, 2,4,5-Trimethylbenzoesäure oder 2,4-6-Trimethylbenzoesäure ist.

4. Verfahren nach Anspruch 1, dadurch gekennzeichnet, daß die Bromionen von Bromwasserstoff, Ammoniumbromid, Natriumbromid oder Alkybromid abgespalten werden.

5. Verfahren nach Anspruch 1, dadurch gekennzeichnet, daß die Bromionen in einer auf das als Lösungsmittel verwendete Wasser bezogenen Menge von 0,5 bis 12 Gew.% verwendet werden.

6. Verfahren nach Anspruch 1, dadurch gekennzeichnet, daß Mangan- oder Cermetallionen höchstens in einer den Bromionen äquivalenten Menge verwendet werden.

7. Verfahren nach Anspruch 1, dadurch gekennzeichnet, daß das Wasser mindestens in derselben Menge verwendet wird wie das polyalkylsubstituierte aromatische Aldehyd oder polyalkylsubstituierte aromatische Säure.

8. Verfahren nach Anspruch 1, dadurch gekennzeichnet, daß die Oxidation bei einer Reaktionstemperatur von 180 bis 280°C und unter einem Überdruck von 15 bis 60kg/cm² durchgeführt wird.

9. Verfahren nach Anspruch 1, dadurch gekennzeichnet, daß der molekulare Sauerstoff in Form von Luft verwendet wird.

10. Verfahren nach Anspruch 1, dadurch gekennzeichnet, daß als Hydrierkatalysator mindestens ein Edelmetall der Gruppe VIII des Periodensystems verwendet wird.

11. Verfahren nach Anspruch 10, dadurch gekennzeichnet, daß die Edelmetalle Pd, Pt, Ru und Rh sind.

12. Verfahren nach Anspruch 1, dadurch gekennzeichnet, daß der Hydrierkatalysator in Form einer einfachen Substanz, einer Legierung oder eines Gemisches oder auf einem Träger verwendet wird.

13. Verfahren nach Anspruch 12, dadurch gekennzeichnet, daß der Träger aus Aktivkohle in Form eines Granulats besteht.

14. Verfahren nach Anspruch 1, dadurch gekennzeichnet, daß die Hydrierung bei 100 bis 200°C und unter einem Druck von 5 bis 30kg/cm² 0, 1 bis 8 Stunden lang durchgeführt wird.

## Revendications

1. Procédé pour préparer un acide aromatique polycarboxylique d'une pureté supérieure à 99%, Ledit procédé, qui comprend l'oxydation d'un aldéhyde aromatique polyalkyl-substitué ou d'un acide carboxylique aromatique polyalkyl-substitué avic de l'oxygène moléculaire dans de l'eau en tant que solvant en présence d'ion bromure ou d'ion bromure et d'ion d'un métal lourd en tant que catalyseur, étant caractérisé en ce que le produit de la réaction d'oxydation est mis en contact avec de l'hydrogène moléculaire en présence d'un catalyseur d'hydrogénation et en l'absence de substance alcaline à une température de 100° à 180°C, le mélange obtenu par la réaction d'hydrogénation est soumis à une distillation sous une pression inférieure à la pression atmosphéque.

2. Procédé suivant la revendication 1, dans lequel l'aldéhyde aromatique polyalkyl-substitué est le 2,4-diméthyl-benzaldéhyde, le 3,4-diméthylbenzaldéhyde, le 2,4,5-triméthylbenzaldéhyde ou le 2,4,6-triméthybenzaldéhyde.

3. Procédé suivant la revendication 1, dans lequel l'acide aromatique polyalkl-substitué est l'acide 2,4-diméthyl-benzoique, l'acide 3,4-diméthylbenzoïque, l'acide 2,4,5-triméthylbenzoïque, ou l'acide 2,4,6-triméthylbenzoïque.

4. Procédé suivant la revendication 1, dans lequel l'ion bromure provient de HBr, $NH_4Br$, NaBr ou d'un bromure d'alkyle.

5. Procédé suivant la revendication 1, dans lequel l'ion bromure est utilisé en quantité de 0, 5 à 12% en poids par rapport à l'eau utilisée en tant que solvant.

6. Procédé suivant la revendication 1, dans lequel l'ion métallique est un ion manganèse ou cérium utilisé en quantité équivalente ou inférieure à celle de l'ion bromure.

7. Procédé suivant la revendication 1, dans lequel l'eau est utilisée selon une quantité pondérale égale ou supérieure à celle de l'aldéhyde aromatique polyalkyl-substitué ou de l'acide aromatique polyalkyl-substitué.

8. Procédé suivant la revendication 1, dans lequel l'oxydation est effectuée à une température de 180°C—280°C, sous une pression de 15 à 60kg/cm².

9. Procédé suivant la revendication 1, dans lequel la source d'oxygene moléculaire est l'air.

10. Procédé suivant la revendication 1, dans lequel le catalyseur d'hydrogénation est au moins un des métaux nobles du groupe 8 de la classification périodique.

11. Procédé suivant la revendication 10 dans lequel les métaux nobles sont Pd, Pt, Ru et Rh.

12. Procédé suivant la revendication 1, dans lequel le catalyseur d'hydrogénation est sous la forme d'une simple substance, d'un alliage, d'un mélange ou associé à un véhicule support.

13. Procédé suivant la revendication 12 dans lequel le véhicule support est le charbon activé sous forme de pastilles.

14. Procédé suivant la revendication 1, dans lequel la réaction d'hydrogénation est effectuée à 100—200°C sous une pression de 5—30kg/cm² pendant 0,1—8h.